# EUROPEAN PATENT SPECIFICATION

(11) **EP 3 296 904 B1**
(45) Date of publication and mention of the grant of the patent: **29.07.2020**
(21) Application number: 17194186.7
(22) Date of filing: 20.12.2012
(51) Int. Cl.: G16H 30/40

(54) **MEDICAL APPARATUS AND IMAGE DISPLAYING METHOD USING THE SAME**
MEDIZINISCHE VORRICHTUNG UND BILDANZEIGEVERFAHREN DAMIT
APPAREIL MÉDICAL ET PROCÉDÉ D'AFFICHAGE D'IMAGE L'UTILISANT

(30) Priority: 21.12.2011 KR 20110139025
(43) Date of publication of application: 21.03.2018
(62) Divisional of application: 12198718.4
(73) Proprietor: Samsung Electronics Co., Ltd., Gyeonggi-do 16677 (KR)
(72) Inventor: LEE, Byeong Won, Gyeonggi-do (KR)
(74) Representative: Grootscholten, Johannes A.M.

(56) References cited:
- WO-A2-2008/064120
- US-A1- 2002 094 119
- US-A1- 2007 140 536
- "PhotoImpact 10 User Guide" In: "PhotoImpact 10 User Guide", 1 August 2004 (2004-08-01), Ulead Systems Inc., XP055057026, pages 1-366, * page 30 - page 32 * * page 42 - page 45 * * page 52 - page 53 * * page 59 * * page 87 - page 96 *

## Description

### BACKGROUND

### 1. Field

Exemplary embodiments relate to a medical apparatus which displays images processed through image processing, and an image display method using the same.

### 2. Description of the Related Art

In general, clinical diagnosis in medical behavior is a major part of treatment of a patient, development of medical techniques facilitates precise clinical diagnosis, and the degree of dependence thereon will continue to increase in the future.

Therefore, medical image equipment, such as a computer tomography (CT) apparatus, a magnetic resonance imager (MRI), an X-ray apparatus and an ultrasonic apparatus, have become essential equipment in modern medical science.

However, since a medical service allows an abnormal region of a patient to be photographed using medical image equipment and then printed on a film. The film must be transmitted to a doctor in charge of the patient. Thus, the medical service has an ineffective structure in that it takes considerable time and a lot of manpower to finally make a clinical diagnosis and causes difficulty in performing rapid and precise treatment of the patient.

Further, as computer and data communication technology develop, a system of providing a medical service using the computer and data communication technology has been researched and developed in the medical community. For example, a medical image system in which a computer communication network is installed throughout the entirety of a hospital, all X-ray films are converted into digital data, a database of the digital data is stored in a large storage medium connected to a server, and an X-ray image of a desired patient is searched through a computer monitor in each medical office as needed, has been introduced.

In order to provide better quality images by removing artifacts, generated due to the physical structure of the medical image equipment, from images acquired through medical image equipment, image processing is needed. US-2002/0094119 discloses manual processing by trained technicians to provide a single processed image in addition to an original analogue image from film, for processing medical images and making adjustments in terms of brightness, contrast, gamma, etc) with the purpose of detection of lesions, tumors, etc, and separate image processing. Additionally, reference is made here to WO-2011/044295, which allows basic image transformations, thumbnails for previews, for radiology (mammography).

Since such image processing by medical staff is important to more precisely and rapidly make a diagnosis, development of a user interface used to perform image processing more conveniently and intuitively is required, and the above acknowledged prior art is susceptible for improvement in these respects.

In addition, US-2007/140536 discloses a method for processing digital x-ray images to maximize diagnostic information including accessing a raw digital image signal generated by imaging a patient with an imaging modality and transmitting the raw image to an acquisition workstation. The acquisition workstation classifies the image and assigns a plurality of image processing conditions. The raw digital signal is processed according to each processing condition. The plurality of processed images are transmitted to a display workstations for review.

### SUMMARY

The invention is set out in the appended independent claims. Advantageous embodiments are defined by the dependent claims.

### BRIEF DESCRIPTION OF THE DRAWINGS

These and/or other aspects of the exemplary embodiments will become apparent and more readily appreciated from the following description of the embodiments, taken in conjunction with the accompanying drawings of which:
FIGS. 1 and 2 are views illustrating the configuration of a medical image system according to an aspect of an exemplary embodiment;
FIGS. 3 and 4 are views illustrating the configuration of a user interface according to an aspect of an exemplary embodiment;
FIG. 5 is a view illustrating the user interface according to an aspect of an exemplary embodiment; and
FIG. 6 is a flowchart illustrating an image displaying method according to an aspect of an exemplary embodiment.

### DETAILED DESCRIPTION OF EXEMPLARY EMBODIMENTS

Reference will now be made in detail to the exemplary embodiments, which are illustrated in the accompanying drawings, wherein like reference numerals refer to like elements throughout the description.

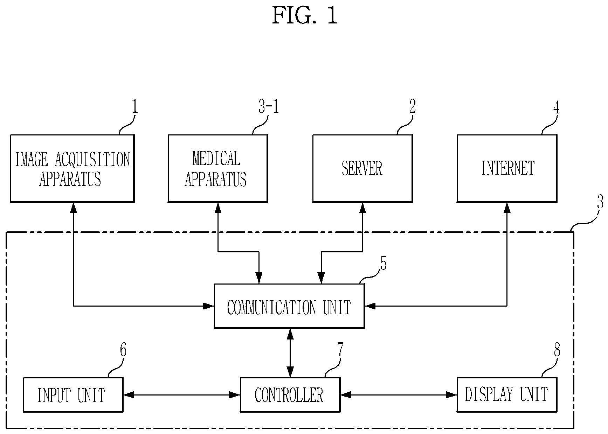

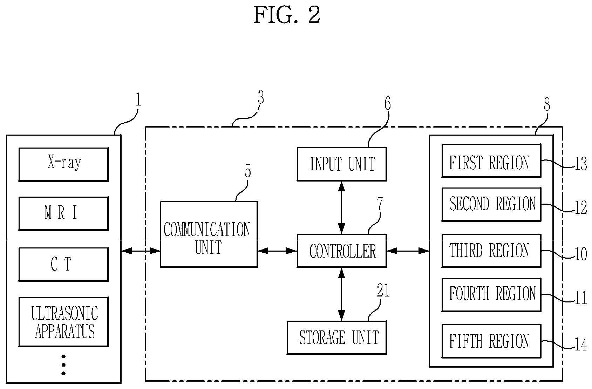

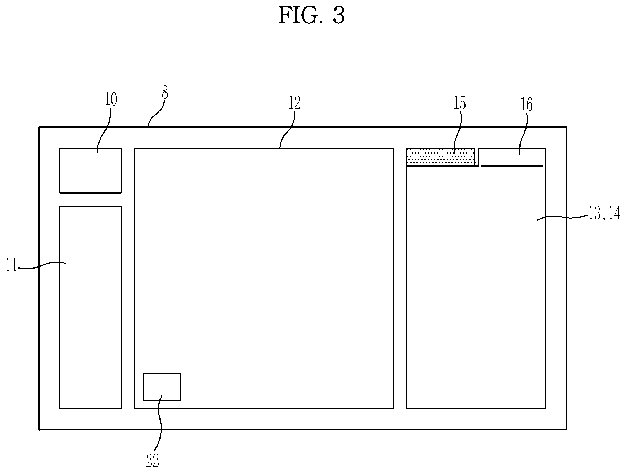

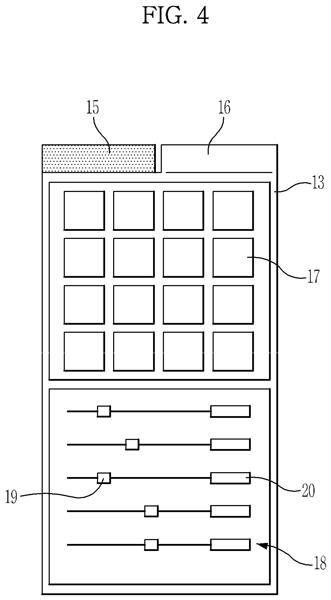

FIGS. 1 and 2 are views illustrating the configuration of a medical image system according to an aspect of an exemplary embodiment, and FIGS. 3 and 4 are views illustrating the configuration of a user interface according to an aspect of an exemplary embodiment.

As shown in FIG. 1, the medical image system includes an image acquisition apparatus 1 acquiring an image of a subject, for example, a patient, and a medical apparatus 3 processing the image processed by the image acquisition apparatus 1 and displaying the processed image.

The medical apparatus 3 is a workstation controlling the overall operation of the medical image system, processes the image processed by the image acquisition apparatus 1, and displays the processed image.

Further, the medical apparatus 3 may be a picture archiving communication system (PACS) viewer which receives the image processed by the image acquisition apparatus 1 from a server 2 or receives the image directly from the image acquisition apparatus 1, processes the received image, and displays the processed image.

The medical apparatus 3 includes a communication unit 5 (e.g., a communicator, transceiver, receiver, receiving unit, etc.) receiving image information of the subject from the image acquisition apparatus 1 through communication with the image acquisition apparatus 1, an input unit 6 providing an interface for operation of the medical apparatus 3, a controller 7 controlling the overall operation of the medical image system including the medical apparatus 3 and the image acquisition apparatus 1 according to instructions input through the input unit 6 and processing the image of the subject received from the communication unit 5, a storage unit 21 storing the processed image and various pieces of information, and a display unit 8 (e.g., a display, etc.) displaying the state of the medical apparatus 3 or the image acquisition apparatus 1 and the various pieces of information and displaying the image processed by the controller 7.

Such a medical apparatus 3 may include a PC, a portable smart-phone or a tablet PC, and a PACS viewer supporting a digital imaging and communication in medicine (DICOM) protocol.

As shown in FIG. 2, the image acquisition apparatus 1 acquires the image of a subject, for example, a patient, and is an MRI, a CT apparatus, an X-ray apparatus or an ultrasonic apparatus. The image of the subject may be at least one from among an MRI image, a CT image, an X-ray image, and an ultrasonic image.

The image acquisition apparatus 1 acquires the image of the subject, and particularly, acquires the image of the inside of the subject, according to the function thereof.

Image data of the subject may be based on the digital imaging and communication in medicine (DICOM) protocol which is a standard protocol.

Although this embodiment describes the communication unit 5 as receiving the image of the subject from the image acquisition apparatus 1, the communication unit 5 may receive various medical images from a server 2 in figure 1 in which images processed by various image acquisition apparatuses are sorted and stored, or may receive medical images via the Internet 4 or from other computers or other medical apparatuses 3-1.

Further, the communication unit 5 may transmit control instructions to control the operation of the image acquisition apparatus 1 from the controller 7 to the image acquisition apparatus 1, or may transmit various pieces of information modified or processed by the controller 7 to the server 2, the Internet 4, or other medical apparatuses 3.

Here, the server 2 may be a PACS server supporting the DICOM protocol.

The communication unit 5 may employ various known wired and wireless communication methods in communication with external devices.

The input unit 6 provides the interface so as to allow a user of the medical apparatus 3, for example, medical staff, to operate the overall function of the medical image system including the medical apparatus 3 and the image acquisition apparatus 1, and may include a remote controller, a mouse, a keyboard, a speech recognition device or a motion recognition device.

The medical staff may properly operate the overall function of the medical image system, as needed, through peculiar functions of the above-described various input devices.

If the display unit 8 uses a touchscreen, a user may operate the function of the medical apparatus 3 by directly touching various icons displayed on the display unit 8. That is, the display unit 8 may perform the function of the input unit 6 in addition to the function of displaying various pieces of information.

The display unit 8 provides a user interface, and the user interface may include a plurality of regions in which windows of various sizes and shapes having peculiar functions are displayed.

The user interface according to an aspect of an exemplary embodiment provides information regarding processing of the image of a subject, i.e., image processing, and provides environments allowing a user to perform operations related to image processing.

With reference to FIGS. 2 and 3, the user interface provided by the display unit 8 includes a first region 13 displaying objects 17 of respective images processed through various predetermined kinds or types of image processing, a second region 12 displaying an image processed through image processing, a third region 10 displaying information regarding the image of a subject, a fourth region 11 displaying a list of selectable image files, and a fifth region 14 providing image processing tools to process the image displayed in the second region 12.

The third region 10 may display the information regarding the subject. On the assumption that the subject is a patient, the third region 10 displays information, such as personal information and disease history of the patient, so that a user may refer to such information.

The fourth region 11 may display a list of image files viewable by the user. The fourth region 11 may display images transmitted from other medical apparatuses 3, the Internet 4 and the server 2, as described above, as well as the image transmitted from the image acquisition apparatus 1.

In the fourth region 11, the images are classified based on the devices or media transmitting the images, and may thus be sorted and displayed according to a device or medium selected by the user.

When the user touches or clicks an image file desired to view from among the image files displayed on the list displayed in the fourth region 11, the corresponding image file is displayed in the fourth region 11, and thus the user may confirm the image of the selected file.

The fifth region 14 displays the image processing tools so as to allow the user to process the image displayed in the second region 12 as desired.

For example, the fifth region 14 may display a tool for adjusting the size of an image displayed on the display unit 8, a tool for rotating the image, a tool for cutting out a desired portion of the image, a tool for magnifying or reducing a desired portion of the image, etc.

These tools may be expressed by symbolized icons intuitively representing the functions of the tools. If the display unit 8 uses a touchscreen, the user may touch the icon expressing a designated tool or click the icon through the input unit 6, thus being capable of processing the image according to the function of the corresponding tool. When the image is processed by the tool selected by the user, the processed image is displayed in the second region 12.

The first region 13 may be designed to possess the same area on the user interface in common with the fifth region 14, and the user may select the first region 13 or the fifth region 14 providing desired contents through tabs 15 and 16 to select the first region 13 and the fifth region 14, displayed on the upper portion of the corresponding area. Although the first region 13 and the fifth region 14 may be displayed in separate areas, since the first region 13 and the fifth region 14 provide contents executing similar functions for the purpose of image processing, the first region 13 and the fifth region 14 may be displayed in the same area so as to allow the user to more easily understand the configuration of the interface. Further, the tabs 15 and 16 are provided so as to allow the user to select the contents displayed in the corresponding area, and thus the user interface may be designed to have a simpler structure.

The second region 12 displaying the image processed through image processing and the first region 13 displaying objects 17 of respective images processed through various predetermined kinds of image processing will be described later with reference to image processing.

The controller 7 controls the overall operation of the medical image system including the medical apparatus 3 and the image acquisition apparatus 1 according to information received through the communication unit 5 or external instructions input through the input unit 6 or the display unit 8.

The controller 7 may control the display unit 8 so as to display a medical image or a diagnosis record, and, if the medical image or the diagnosis record is amended or updated, may store the amended or updated medical image or diagnosis record in the storage unit 21, and may transmit the amended or updated medical image or diagnosis record to the server 2 through the communication unit 5 so as to store the amended or updated medical image or diagnosis record in the server 2.

The controller 7 basically performs image processing to provide an image having a more improved quality, when the controller 7 receives the image of the subject transmitted from the image acquisition apparatus 1 through the communication unit 5.

Image processing may be divided into pre-processing and post-processing.

Pre-processing is a process of removing artifacts or errors generated due to the physical structure of the image acquisition apparatus 1, and post-processing is a process of improving the quality of an image having undergone pre-processing and during post-processing, contrast, sharpness and brightness are determined according to set parameters.

The controller 7 displays the image processed through image processing on the display unit 8. The image having undergone the basic image processing is displayed in the second region 12 of the user interface provided by the display unit 8.

Separately from the above-described image processing basically executed with respect to the received image, the controller 7 executes various predetermined kinds of image processing with respect to the received image. More specifically, the controller 7 executes various kinds of post-processing of the image having undergone pre-processing.

For example, the controller 7 executes various kinds of post-processing exhibiting various effects, such as brightness, contrast, sharpness, saturation, film-effect, window/level, etc., with respect to the image from which the artifacts or errors are removed through pre-processing. Hereinafter, the above-described basic image processing will be referred to as first image processing, and the various kinds of predetermined image processing executed separately from the first image processing will be referred to as second image processing.

That is, when the controller 7 receives an image, the controller 7 amends artifacts or errors of the image through pre-processing, executes first image processing by executing first post-processing with respect to the image having undergone pre-processing, and executes second image processing by respectively executing the above-described various kinds of second post-processing with respect to the image having undergone pre-processing. The various kinds of second post-processing may include all kinds of post-processing which may be executed with respect to the image. Further, the controller 7 may select kinds of post-processing which a user often uses by calculating the use frequencies of the kinds of post-processing selected by the user, and may form second post-processing consisting of the selected kinds of post-processing.

The controller 7 generates images variously processed by executing second image processing, and displays objects 17 of the images in the first region 13 of the display unit 8.

Here, the objects 17 include thumbnail pictures of the images processed by executing various kinds of post-processing and texts expressing features of the respective kinds of post-processing. The text representing the features of a kind of post-processing means, for example, if the kind of post-processing applied to the image is change of contrast, a text expressing the feature of the corresponding kind of post-processing, such as contrast, of the image having undergone the corresponding kind of post-processing. In order to allow a user to intuitively recognize and expect an execution result of post-processing, the objects 17 may be expressed by thumbnail pictures or expressed by both thumbnail pictures and texts.

Second image processing together with first image processing is executed with respect to the image having undergone pre-processing. Differently from first image processing, in second image processing, various kinds of post-processing are respectively executed and various images processed by executing the respective kinds of post-processing are generated, as described above, and thus second image processing may take a longer time than first image processing.

When first image processing is completed, an image processed by executing first image processing is displayed in the second region 12 of the user interface, and if second image processing is not completed, the thumbnail picture of the image is not displayed. Therefore, until second image processing is completed, the objects 17 are expressed by texts in the first region 13, and when second image processing is completed, the texts may be changed to thumbnail pictures, or both the texts and the thumbnail pictures may be displayed. Of course, when second image processing is completed simultaneously with first image processing or is completed prior to first image processing, the objects 17 may be expressed by thumbnail pictures.

Since the respective objects 17 represent images to which various kinds of post-processing are respectively applied, when a user inputs instructions to an object 17, a processed image represented by the object 17 to which the instructions are input is magnified and displayed on the display unit 8.

For example, if a user desires to change the contrast of the image displayed in the second region 12, when the user touches or clicks the thumbnail picture of the image in which contrast is changed from among several thumbnail pictures 17, the image represented by the corresponding thumbnail picture is magnified and displayed in the second region 12.

In this way, when images processed by executing various kinds of post-processing are represented by the objects 17 allowing a user to intuitively recognize kinds of post-processing to be executed, such as the thumbnail pictures, the user may touch or click a thumbnail picture to confirm an image to which the corresponding kind of post-processing is applied.

The user may only touch or click a thumbnail picture while confirming an image processed by executing a kind of post-processing regarding a corresponding parameter and expressed by the thumbnail picture in advance without a troublesome operation, such as a manual adjustment of the parameter, and thus may more easily and simply process the image as the user desires.

From the viewpoint of the user interface, the second region 12 displays an image to which first image processing is applied, and the first region 13 displays thumbnail pictures of respective images to which second image processing is applied. When the tab 16 to select the first region 13 from among the first region 13 and the fifth region 14 providing processing tools to process an image, displayed in the same area is touched or clicked, contents provided by the first region 13 is displayed in the corresponding area. That is, the first region 13 displays thumbnail pictures of respective images to which second image processing is applied. When a thumbnail picture to which a desired kind of post-processing is applied is touched or clicked from among these several thumbnail pictures, a processed image represented by the thumbnail picture is magnified and displayed in the second region 12.

A window 22 displaying an image before it is changed into a processed image may be provided in the second region 12, and when the window 22 is touched or clicked, the image before the change into the processed image may be displayed again. That is, the image before the change and the image after the change may be compared and be converted directly therebetween. The window 22 displaying the image before the change may be provided to occupy a small-sized area at the corner of the second region 12 if display of the image after the change is emphasized, and may be provided to occupy the same-sized area or a similar-sized area as or to the image after the change if a comparison between the image before the change and the image after the change is emphasized. This may be adjusted according to a change of setting by a user.

A parameter adjustment window 18 may be provided in the first region 13 so as to be manipulated when a user desires more detailed post-processing. The user may input specific parameter values into value input windows 20 displayed in the parameter adjustment window 18, or may drag icons 19 to adjust parameters.

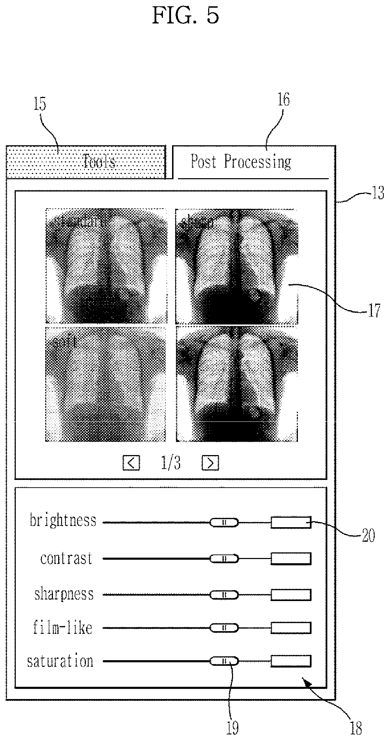

FIG. 5 is a view illustrating the user interface according to an aspect of an exemplary embodiment.

FIG. 5 specifically illustrates the above-described first region 13. When the tab 16 to select the first region 13 is touched or clicked from among the tabs 15 and 16 to select one of the first region 13 and the fifth region 14, contents provided by the first region 13 may be displayed, as shown in FIG. 5.

Thumbnail pictures 17 and texts of images processed by executing various predetermined kinds of image processing, i.e., second image processing, are displayed at the upper portion of the first region 13. As described above, the user may touch or click the thumbnail picture 17 to which a desired kind of image processing is applied from among the displayed thumbnail pictures 17, thus confirming the processed image represented by the corresponding thumbnail picture 17 in the second region 12.

The parameter adjustment window 18 may be displayed at the lower portion of the first region 13 so as to be manipulated when the user desires more detailed post-processing. The user may input specific parameter values into the value input windows 20 displayed in the parameter adjustment window 18, or may drag the icons 19 to adjust parameters.

Although the user interface of FIG. 5 is an example of FIG. 4, the exemplary embodiments not limited thereto and the user interface of FIG. 5 may be modified in various ways.

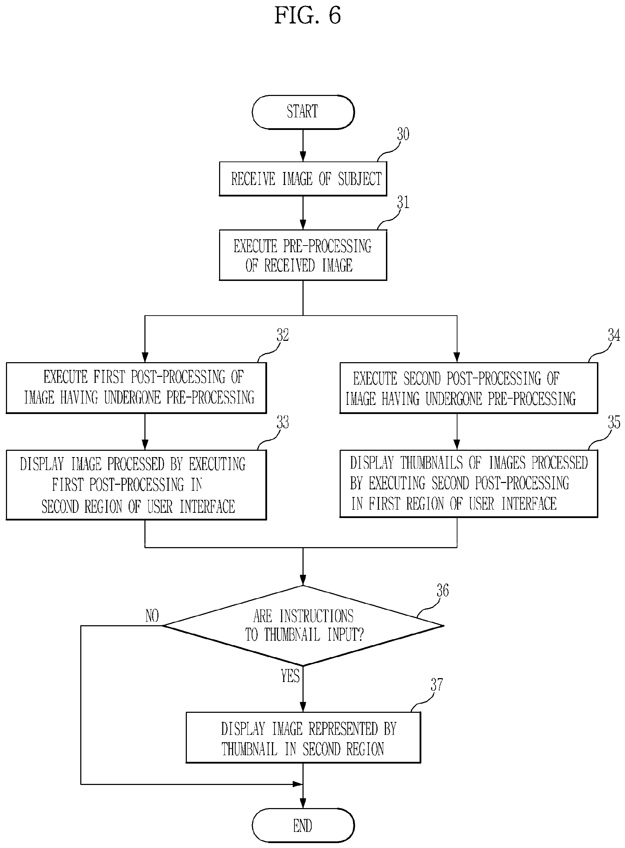

FIG. 6 is a flowchart illustrating an image displaying method according to an aspect of an exemplary embodiment.

With reference to FIG. 6, first, an image of a subject is received (Operation 30).

The communication unit 5 receives the image of the subject from the image acquisition apparatus 1, another medical apparatus 3, the Internet 4 or the server 2.

When the image of the subject is received, the controller 7 executes pre-processing of the received image (Operation 31). Pre-processing is a process of removing artifacts or errors generated due to the physical structure of the image acquisition apparatus 1, and is basically executed with respect to the received image.

When pre-processing of the image of the subject is completed, the controller 7 executes first post-processing of the image, pre-processing of which is completed (Operation 32).

Pre-processing and first post-processing are image processing basically executed to improve the quality of the received image, and correspond to the above-described first image processing.

When first post-processing is completed, the controller 7 displays the image processed by executing the first post-processing in the second region 12 of the user interface (Operation 33).

Further, when pre-processing of the image of the subject is completed, the controller 7 executes second post-processing of the image, pre-processing of which is completed (Operation 34).

As described above, the controller 7 processes the image, pre-processing of which is completed, by executing various kinds of post-processing and adjusting various effects, such as brightness, contrast, sharpness, saturation, film-effect, window/level, etc. Therefore, images exhibiting different effects are generated according to types of applied post-processing.

When second post-processing is completed, the controller 7 generates thumbnail pictures of the respective images and displays the generated thumbnail pictures in the first region 13 of the user interface (Operation 35). Operations 34 and 35 may be executed simultaneously with Operations 32 and 33. Only the thumbnail pictures may be displayed, or the thumbnail pictures together with texts representing features of the kinds of post-processing may be displayed.

The image to which first post-processing is applied is displayed in the second region 12, and the thumbnail pictures of the images to which kinds of second post-processing are applied are displayed in the first region 13. When instructions to a thumbnail picture displayed in the first region 13 are input (Operation 36), the controller 7 magnifies a processed image represented by the thumbnail picture and displays the processed image in the second region 12 (Operation 37).

The instructions to the thumbnail picture may be input by touching the thumbnail picture if the display unit 8 employs a touchscreen, or be input by clicking the thumbnail picture through the input unit 6 including a remote controller, a mouse, a keyboard, a speech recognition device or a motion recognition device.

When the thumbnail picture is touched or clicked in this way, the processed image represented by the thumbnail picture is magnified and displayed in the second region 12. For example, when a user touches or clicks the thumbnail picture of an image formed by changing contrast from among the several thumbnail pictures displayed in the first region 13, the image represented by the corresponding thumbnail picture is magnified and displayed in the second region 12. Here, magnification does not mean exaggeration of a specific part but means fitting of the image represented by the thumbnail to the size of the second region 12.

The medical apparatus according to an aspect of an exemplary embodiment provides the user interface displaying thumbnail pictures of images to which various types of post-processing are applied in advance. When a user desires to apply a desired effect to an image displayed on the display unit 8, the user may process the image by touching or clicking a thumbnail picture exhibiting the desired effect from among various thumbnail pictures in which various types of post-processing are executed, without direct change of parameters or direct input of values. Since an image exhibiting the desired effect may be intuitively recognized and selected through the thumbnail picture, the user may more easily and simply process the image.

As is apparent from the above description, in a medical apparatus and an image display method using the same according to an aspect of an exemplary embodiment, various post-processing results of an image may be intuitively recognized and selected. Therefore, a user may more rapidly and conveniently process the image and thus make a more correct clinical diagnosis.

Although a few exemplary embodiments have been shown and described, it would be appreciated by those skilled in the art that changes may be made in the exemplary embodiments without departing from the principles of the inventive concept, the scope of which is defined in the claims.

## Claims

1. A medical image processing apparatus comprising:
a receiver configured to receive an image of a subject, which image is obtained with a medical image acquisition apparatus from a group comprising: computer tomography (CT) apparatus; a magnetic resonance imager (MRI); an X-ray apparatus; and an ultrasonic apparatus;
a controller configured to process the image through multiple predetermined types of image processing, when the image of the subject is received, and to generate objects corresponding to the processed image; and
a display (8) configured to display generated objects corresponding to the processed image,
wherein the controller is configured to process the image separately through a first type image processing and through a plurality of second types of image processing, and, when second types of image processing are completed, to generate multiple thumbnail objects (17) of the image processed through the second types of image processing,
wherein the display (8) is controlled to display the multiple thumbnail objects (17) in a first region (13) and the first type processed image in a second region (12),
wherein the controller is configured to further accept user input, such as a touch or click, of a selection of one of the multiple thumbnail objects (17) for magnified display in the second region (12) of the image processed through one of the second types of image processing represented by the selected one of the multiple thumbnail objects (17) and for fitting the image processed through one of the second types of image processing represented by the selected one of the multiple thumbnail objects (17) to a size of the second region (12).

2. The medical image processing apparatus according to claim 1, wherein the thumbnail objects (17) comprise thumbnail pictures of the image processed through the second types of image processing and/or text representing features of the second types of image processing.

3. The medical image processing apparatus according to claim 1 or 2, wherein the the controller is configured to determine kinds of image processing, which a user often uses, by calculating the use frequencies of the kinds of image processings selected by said user, and to select said kinds of image processing as said plurality of second types of image processing.

4. The medical image processing apparatus according to claim 1 or 2, wherein the display is configured to display the image of the subject, information regarding the image, a list of viewable images, tools to process the image and the thumbnail objects (17) of the image processed by the second types of image processing, and to display if instructions are input to magnify in the second region (12) the selected thumbnail object (17) displayed in the first region (13), the image resulting from the one of the second types of image processing represented by the thumbnail object (17).

5. A control method of a medical image processing apparatus, comprising:
receiving an image of a subject, which image is obtained with a medical image acquisition apparatus from a group comprising: computer tomography (CT) apparatus; a magnetic resonance imager (MRI); an X-ray apparatus; and an ultrasonic apparatus;
processing the image through multiple predetermined types of image processing, when the image of the subject is received; and
generating objects corresponding to the image processed through the multiple types of image processing and displaying objects corresponding to the processed image on a display of the medical image processing apparatus,
processing the image separately through a first type image processing and through a plurality of second types of image processing, and, when second types of image processing are complete, generating multiple thumbnail objects (17) of the image processed through the second types of image processing,
displaying the multiple thumbnail objects (17) in a first region (13) and the first type processed image in a second region (12), and
accepting user input, such as a touch or click, of a selection of one of the multiple thumbnail objects (17) for magnified display in the second region (12) of the image processed through one of the second types of image processing represented by the selected one of the multiple thumbnail objects (17) and for fitting the image processed through one of the second types of image processing represented by the selected one of the multiple thumbnail objects (17) to a size of the second region (12).

6. The control method according to claim 5, wherein the thumbnail objects (17) comprise thumbnail pictures of the image processed through the second types of image processing and/or text representing features of the second types of image processing.

7. The control method according to claim 5 or 6, wherein, when the image of the subject is received, the overall image of the subject is displayed.

8. The control method according to any one of the preceding claims 5 - 7, wherein the thumbnail objects (17) together with the overall image of the subject are displayed on the display of the medical apparatus.

9. The control method according to any one of preceding claims 5 - 8, further comprising:
displaying the thumbnail objects (17) representing the image on the display, during the processing of the image separately from the processing of the image.

10. The control method according to any one of preceding claims 5 - 9, wherein each of the second types of processing adjusts at least one parameter of the image from a group of parameters, comprising brightness, contrast, sharpness, saturation, film-effect and window/level.

11. The control method according to any one of preceding claims 5 - 10, further comprising: determining kinds of image processing, which a user often uses, by calculating the use frequencies of the kinds of image processings selected by said user, and selecting said kinds of image processing as said plurality of second types of image processing.

12. A non-transitory computer readable recording medium comprising a program comprising instructions to cause the medical image processing apparatus of any one of claims 1-4 to execute the steps of any one of the methods of claims 5-11.

## Patentansprüche

1. Medizinische Bildverarbeitungsvorrichtung, die Folgendes umfasst:
einen Empfänger, der zum Empfangen eines Bildes eines Subjekts konfiguriert ist, wobei das Bild mit Hilfe einer Vorrichtung zur Erfassung von medizinischen Bildern aus der folgenden Gruppe erhalten wird: eine Computertomographievorrichtung (CT-Vorrichtung); eine Magnetresonanztomographieeinrichtung (MRT); eine Röntgenvorrichtung; und eine Ultraschallvorrichtung;
eine Steuerung, die dazu konfiguriert ist, das Bild durch mehrere vorbestimmte Arten von Bildverarbeitung zu verarbeiten, wenn das Bild des Subjekts empfangen wird, und dazu, Objekte zu erzeugen, die dem verarbeiteten Bild entsprechen; und
eine Anzeige (8), die dazu konfiguriert ist, die erzeugten, dem verarbeiteten Bild entsprechenden Objekte anzuzeigen,
wobei die Steuerung dazu konfiguriert ist, das Bild separat mit Hilfe einer ersten Art von Bildverarbeitung und mit Hilfe einer Vielzahl von zweiten Arten von Bildverarbeitung zu verarbeiten, und wenn die zweiten Arten von Bildverarbeitung vollendet sind, mehrere Miniaturobjekte (17) des mit Hilfe der zweiten Arten von Bildverarbeitung verarbeiteten Bildes zu erzeugen,
wobei die Anzeige (8) derart gesteuert wird, dass sie die mehreren Miniaturobjekte (17) in einem ersten Bereich (13) anzeigt und das mit Hilfe der ersten Art verarbeitete Bild in einem zweiten Bereich (12) anzeigt,
wobei die Steuerung dazu konfiguriert ist, weiterhin eine Benutzereingabe wie eine Berührung oder einen Klick zur Auswahl eines aus der Vielzahl von Miniaturobjekten (17) für eine vergrößerte Anzeige in dem zweiten Bereich (12) des Bildes, das durch eine der zweiten Arten der Bildverarbeitung verarbeitet wird, die durch das ausgewählte aus der Vielzahl von Miniaturobjekten (17) dargestellt wird, und zum Anpassen des Bildes, das durch eine der zweiten Arten der Bildverarbeitung verarbeitet wurde, die durch das ausgewählte aus der Vielzahl von Miniaturobjekten (17) dargestellt wird, an eine Größe des zweiten Bereichs (12).

2. Medizinische Bildverarbeitungsvorrichtung nach Anspruch 1, wobei die Miniaturobjekte (17) Miniaturansichtsbilder des durch die zweite Art von Bildverarbeitung verarbeiteten Bildes und/oder einen Text, der die Eigenschaften der zweiten Arten von Bildverarbeitung repräsentiert, umfassen.

3. Medizinische Bildverarbeitungsvorrichtung nach Anspruch 1 oder 2, wobei die Steuerung dazu konfiguriert ist, Sorten von Bildverarbeitung zu bestimmen, die ein Benutzer häufig verwendet, durch Berechnen der Häufigkeit einer Verwendung der von dem Benutzer ausgewählten Sorten von Bildverarbeitung, und dazu, diese Sorten der Bildverarbeitung als die Vielzahl von zweiten Arten der Bildverarbeitung auszuwählen.

4. Medizinische Bildverarbeitungsvorrichtung nach Anspruch 1 oder 2, wobei die Anzeige dazu konfiguriert ist, Folgendes anzuzeigen: das Bild des Subjekts, Informationen bezüglich des Bildes, eine Liste von sichtbaren Bildern, Werkzeuge zum Verarbeiten des Bildes und die Miniaturobjekte (17) des Bildes, die mit Hilfe der zweiten Arten von Bildverarbeitung verarbeitet wurden, und dazu konfiguriert ist, wenn Anweisungen eingegeben werden, das im ersten Bereich (13) angezeigte, ausgewählte Miniaturobjekt (17) im zweiten Bereich (12) zu vergrößern, das Bild anzuzeigen, das sich aus der einen der zweiten Arten der Bildverarbeitung ergibt, die durch das Miniaturobjekt (17) dargestellt wird.

5. Steuerungsverfahren für eine medizinische Bildverarbeitungsvorrichtung, wobei das Verfahren Folgendes umfasst:
Empfangen eines Bildes eines Subjekts, wobei das Bild mit Hilfe einer Vorrichtung zur Erfassung von medizinischen Bildern aus der folgenden Gruppe erhalten wird: eine Computertomographievorrichtung (CT-Vorrichtung); eine Magnetresonanztomographieeinrichtung (MRT); eine Röntgenvorrichtung; und eine Ultraschallvorrichtung;
Verarbeiten des Bildes mit Hilfe mehrerer vorbestimmter Arten von Bildverarbeitung, wenn das Bild des Subjekts empfangen wird; und
Erzeugen von Objekten, die dem Bild entsprechen, das mit Hilfe der mehreren Arten von Bildverarbeitung verarbeitet wurde, und Anzeigen der Objekte, die dem verarbeiteten Objekt entsprechen, auf einer Anzeige der medizinischen Bildverarbeitungsvorrichtung,
Verarbeiten des Bildes separat mit Hilfe einer ersten Art von Bildverarbeitung und mit Hilfe einer Vielzahl von zweiten Arten von Bildverarbeitung, und, wenn die zweiten Arten von Bildverarbeitung vollendet sind, Erzeugen mehrerer Miniaturobjekte (17) des mit Hilfe der zweiten Arten von Bildverarbeitung verarbeiteten Bildes,
Anzeigen der mehreren Miniaturobjekte (17) in einem ersten Bereich (13) und des mit Hilfe der ersten Art verarbeiteten Bildes in einem zweiten Bereich (12), und
Annehmen einer Benutzereingabe wie eine Berührung oder einen Klick zur Auswahl eines aus der Vielzahl von Miniaturobjekten (17) für eine vergrößerte Anzeige in dem zweiten Bereich (12) des Bildes, das durch eine der zweiten Arten der Bildverarbeitung verarbeitet wird, die durch das ausgewählte aus der Vielzahl von Miniaturobjekten (17) dargestellt wird, und zum Anpassen des Bildes, das durch eine der zweiten Arten der Bildverarbeitung verarbeitet wurde, die durch das ausgewählte aus der Vielzahl von Miniaturobjekten (17) dargestellt wird, an eine Größe des zweiten Bereichs (12).

6. Steuerungsverfahren nach Anspruch 5, wobei die Miniaturobjekte (17) Miniaturbilder des durch die zweiten Arten von Bildverarbeitung verarbeiteten Bildes und/oder einen Text, der die Eigenschaften der zweiten Arten von Bildverarbeitung repräsentiert, umfassen.

7. Steuerungsverfahren nach Anspruch 5 oder 6, wobei, wenn das Bild des Subjekts empfangen wird, das Gesamtbild des Subjekts angezeigt wird.

8. Steuerungsverfahren nach einem der vorhergehenden Ansprüche 5 bis 7, wobei die Miniaturobjekte (17) gemeinsam mit dem Gesamtbild des Subjekts auf der Anzeige der medizinischen Vorrichtung angezeigt werden.

9. Steuerungsverfahren nach einem der vorhergehenden Ansprüche 5 bis 8, wobei das Verfahren weiterhin Folgendes umfasst:
Anzeigen der Miniaturobjekte (17), die das Bild darstellen, auf der Anzeige während der Verarbeitung des Bildes, getrennt von der Verarbeitung des Bildes.

10. Steuerungsverfahren nach einem der vorhergehenden Ansprüche 5 bis 9, wobei jede der zweiten Arten von Verarbeitung wenigstens einen Parameter des Bildes aus einer Gruppe von Parametern einschließlich Helligkeit, Kontrast, Schärfe, Sättigung, Filmeffekt und Fenster/Ebene anpasst.

11. Steuerungsverfahren nach einem der vorhergehenden Ansprüche 5 bis 10, wobei das Verfahren weiterhin Folgendes umfasst: Bestimmen von Sorten von Bildverarbeitung, die ein Benutzer häufig verwendet, durch Berechnen der Häufigkeit einer Verwendung der von dem Benutzer ausgewählten Sorten von Bildverarbeitung, und Auswählen dieser Sorten der Bildverarbeitung als die Vielzahl von zweiten Arten der Bildverarbeitung.

12. Nicht-flüchtiges, computerlesbaren Aufzeichnungsmedium, das ein Programm umfasst, welches Anweisungen umfasst, um zu bewirken, dass die medizinische Bildverarbeitungsvorrichtung nach einem der Ansprüche 1 bis 4 die Schritte nach einem der Verfahren der Ansprüche 5 bis 11 ausführt.

## Revendications

1. Appareil médical de traitement d'images, comprenant :
un récepteur conçu pour recevoir une image d'un sujet, laquelle image est obtenue à l'aide d'un appareil d'imagerie médicale appartenant au groupe comprenant : un appareil de tomodensitométrie (TDM), un appareil d'imagerie par résonance magnétique (IRM), un appareil de radiographie et un appareil à ultrasons,
un organe de commande conçu pour traiter l'image par le biais de multiples types de traitement d'image prédéterminés, une fois l'image du sujet reçue, et pour produire des objets qui correspondent à l'image traitée, et
un dispositif d'affichage (8) conçu pour afficher les objets produits qui correspondent à l'image traitée ;
ledit organe de commande étant conçu pour traiter l'image séparément par le biais d'un premier type de traitement d'image et par le biais d'une pluralité de seconds types de traitement d'image, et, une fois les seconds types de traitement d'image réalisés, pour produire de multiples objets vignettes (17) de l'image traitée par le biais des seconds types de traitement d'image,
ledit dispositif d'affichage (8) étant commandé pour afficher les multiples objets vignettes (17) dans une première zone (13) et l'image issue du premier type de traitement dans une seconde zone (12),
ledit organe de commande étant conçu pour accepter en outre une saisie réalisée par l'utilisateur, sous la forme d'une entrée tactile ou d'un clic, concernant la sélection d'un objet vignette parmi les multiples objets vignettes (17) afin qu'il soit affiché grossi dans la seconde zone (12) de l'image traitée par le biais d'un des seconds types de traitement d'image représenté par l'objet vignette choisi parmi les multiples objets vignettes (17) et pour adapter l'image, traitée par le biais d'un des seconds types de traitement d'image représenté par l'objet vignette choisi parmi les multiples objets vignettes (17), à la taille de la seconde zone (12).

2. Appareil médical de traitement d'images selon la revendication 1, dans lequel les objets vignettes (17) comprennent des vues au format vignettes de l'image traitée par le biais des seconds types de traitement d'image et/ou du texte représentant les caractéristiques des seconds types de traitement d'image.

3. Appareil médical de traitement d'images selon la revendication 1 ou 2, dans lequel l'organe de commande est conçu pour déterminer les sortes de traitement d'image qu'un utilisateur utilise fréquemment, en calculant les fréquences d'utilisation des sortes de traitements d'image sélectionnées par ledit utilisateur, et pour sélectionner lesdites sortes de traitement d'image comme pluralité desdits seconds types de traitement d'image.

4. Appareil médical de traitement d'images selon la revendication 1 ou 2, dans lequel le dispositif d'affichage est conçu pour afficher l'image du sujet, des informations concernant l'image, une liste d'images visualisables, des outils de traitement de l'image et des objets vignettes (17) de l'image traitée par le biais des seconds types de traitement d'image, et pour afficher, en cas de saisie d'instructions visant à grossir, dans la seconde zone (12), l'objet vignette (17) affiché dans la première zone (13) et ayant été sélectionné, l'image résultant dudit un des seconds types de traitement d'image représenté par l'objet vignette (17).

5. Procédé de commande d'un appareil médical de traitement d'images, comprenant :
la réception d'une image d'un sujet, laquelle image est obtenue à l'aide d'un appareil d'imagerie médicale appartenant au groupe comprenant : un appareil de tomodensitométrie (TDM), un appareil d'imagerie par résonance magnétique (IRM), un appareil de radiographie et un appareil à ultrasons,
le traitement de l'image par le biais de multiples types de traitement d'image prédéterminés, une fois l'image du sujet reçue, et
la production d'objets correspondant à l'image traitée par le biais des multiples types de traitement d'image et l'affichage des objets correspondant à l'image traitée sur un dispositif d'affichage de l'appareil médical de traitement d'images ;
le traitement de l'image séparément, par le biais d'un premier type de traitement d'image et par le biais d'une pluralité de seconds types de traitement d'image, et, une fois les seconds types de traitement d'image réalisés, la production de multiples objets vignettes (17) de l'image traitée par le biais des seconds types de traitement d'image,
l'affichage des multiples objets vignettes (17) dans une première zone (13) et de l'image issue du premier type de traitement dans une seconde zone (12), et
l'acceptation d'une saisie réalisée par l'utilisateur, sous la forme d'une entrée tactile ou d'un clic, concernant la sélection d'un objet parmi les multiples objets vignettes (17) afin qu'il soit affiché grossi dans la seconde zone (12) de l'image traitée par le biais d'un des seconds types de traitement d'image représenté par l'objet vignette choisi parmi les multiples objets vignettes (17) et afin d'adapter l'image, traitée par le biais d'un des seconds types de traitement d'image représenté par l'objet vignette choisi parmi les multiples objets vignettes (17), à la taille de la seconde zone (12).

6. Procédé de commande selon la revendication 5, dans lequel les objets vignettes (17) comprennent des vues au format vignettes de l'image traitée par le biais des seconds types de traitement d'image et/ou du texte représentant les caractéristiques des seconds types de traitement d'image.

7. Procédé de commande selon la revendication 5 ou 6, dans lequel, une fois l'image du sujet reçue, l'image globale du sujet est affichée.

8. Procédé de commande selon l'une quelconque des revendications précédentes 5 à 7, dans lequel les objets vignettes (17) sont affichés conjointement à l'image globale du sujet sur le dispositif d'affichage de l'appareil médical.

9. Procédé de commande selon l'une quelconque des revendications précédentes 5 à 8, comprenant en outre :
l'affichage, sur le dispositif d'affichage, des objets vignettes (17) représentant l'image, au cours du traitement de l'image séparément du traitement de l'image.

10. Procédé de commande selon l'une quelconque des revendications précédentes 5 à 9, dans lequel chacun des seconds types de traitement ajuste au moins un paramètre de l'image parmi un groupe de paramètres, dont la luminosité, le contraste, la netteté, la saturation, les effets sur le cliché et le niveau de fenêtre.

11. Procédé de commande selon l'une quelconque des revendications précédentes 5 à 10, comprenant en outre : la détermination des sortes de traitement d'image qu'un utilisateur utilise fréquemment, en calculant les fréquences d'utilisation des sortes de traitements d'image sélectionnées par ledit utilisateur, et la sélection desdites sortes de traitement d'image comme pluralité desdits seconds types de traitement d'image.

12. Support d'enregistrement non transitoire lisible par ordinateur, comprenant un programme comprenant des instructions visant à amener l'appareil médical de traitement d'images selon l'une quelconque des revendications 1 à 4 à exécuter les étapes de l'un quelconque des procédés selon les revendications 5 à 11.
